# EUROPÄISCHE PATENTSCHRIFT

(11) **EP 0 832 870 B1**
(45) Veröffentlichungstag und Bekanntmachung des Hinweises auf die Patenterteilung: **25.07.2001**
(21) Anmeldenummer: 97115589.0
(22) Anmeldetag: 09.09.1997
(51) Int. Cl.: C07C 45/67, C07C 49/603

(54) **Verfahren zur Herstellung von 3,5,5-Trimethylcyclohexa-3-en-1-on (Beta-Isophoron) durch Isomerisierung von 3,5,5-Trimethycyclohexa-2-en-1-on (Alpha-Isophoron)**
Process for the preparation of 3,5,5-trimethylcyclohex-3-ene-1-one (beta-isophoron) by isomerisation of 3,5,5-trimethylcyclohex-2-ene-1-one (alpha-isophoron)
Procédé pour la préparation de 3,5,5-triméthylcyclohex-3-ène-1-one (beta-isophorone) par isomérisation de 3,5,5-triméthylcyclohex-2-ène-1-one (alpha-isophorone)

(30) Priorität: 26.09.1996 DE 19639570
(43) Veröffentlichungstag der Anmeldung: 01.04.1998
(73) Patentinhaber: Degussa AG, 40474 Düsseldorf (DE)
(72) Erfinder: Krill, Steffen, Dr., 67346 Speyer (DE); Giray, Günes, Mavischir-Didim (TR); Huthmacher, Klaus, Dr., 63571 Gelnhausen (DE); Hübner, Frank, Dr., 64372 Ober-Ramstadt (DE); Tanner, Herbert, Dr., 63457 Hanau-Wolfgang (DE)

(56) Entgegenhaltungen:
- EP-A- 0 488 045
- FR-A- 2 074 410
- PATENT ABSTRACTS OF JAPAN vol. 013, no. 457 (C-644), 16.Oktober 1989 & JP 01 175954 A (SODA KORYO KK), 12.Juli 1989,

## Beschreibung

Die Erfindung betrifft ein Verfahren zur Herstellung von 3,5,5-Trimethylcyclohexa-3-en-1-on (β-Isophoron) durch Isomerisierung von 3,5,5-Trimethylcyclohexa-2-en-1-on (α-Isophoron) in der Flüssigphase in Gegenwart eines heterogenen Katalysators.

β-Isophoron hat großes wirtschaftliches Interesse, da es einen wichtigen Synthesebaustein für die Herstellung von Carotinoiden, Vitaminen und pharmazeutischen Produkten darstellt. Insbesondere wird β-Isophoron als Vorstufe für Ketoisophoron (= 2,6,6-Trimethylcyclohex-2-en-1,4-dion) und Trimethylhydrochinon und damit für die Herstellung von Vitamin E benötigt. Daneben geht es zentral in Synthesen für Riechstoffe und Naturstoffe wie Astaxanthin und Abscisinsäure und Derivate ein.

Die Herstellung von Isophoron gelingt durch Aceton-Trimerisierung, unter Kondensation der C₃-Bausteine. Das hauptsächlich gebildete Isomere ist das α-Isophoron, da es im Gegensatz zum β-Isomeren eine zur Ketofunktion konjugierte Doppelbindung besitzt. Aus diesem Grund liegt das thermodynamische Gleichgewicht auf Seiten des α-Isophorons; die β-Konzentration beträgt nur etwa 1 - 2 % und die Gleichgewichtseinstellung erfolgt sehr langsam.

Obwohl es grundsätzlich zwei verschiedenen Ansätze gibt, zum Ketoisophoron zu gelangen, nämlich die Direktoxidation von α-Isophoron → Ketoisophoron und dem Umweg über die Isomerisierung α-Isophoron → β-Isophoron in einem Primärschritt und anschließende Oxidation des β-Isophoron → Ketoisophoron, ist letzteres Verfahren deutlich vorteilig. Schema 1 stellt diese Überlegungen zur Ketoisophoron-Synthese anschaulich dar: 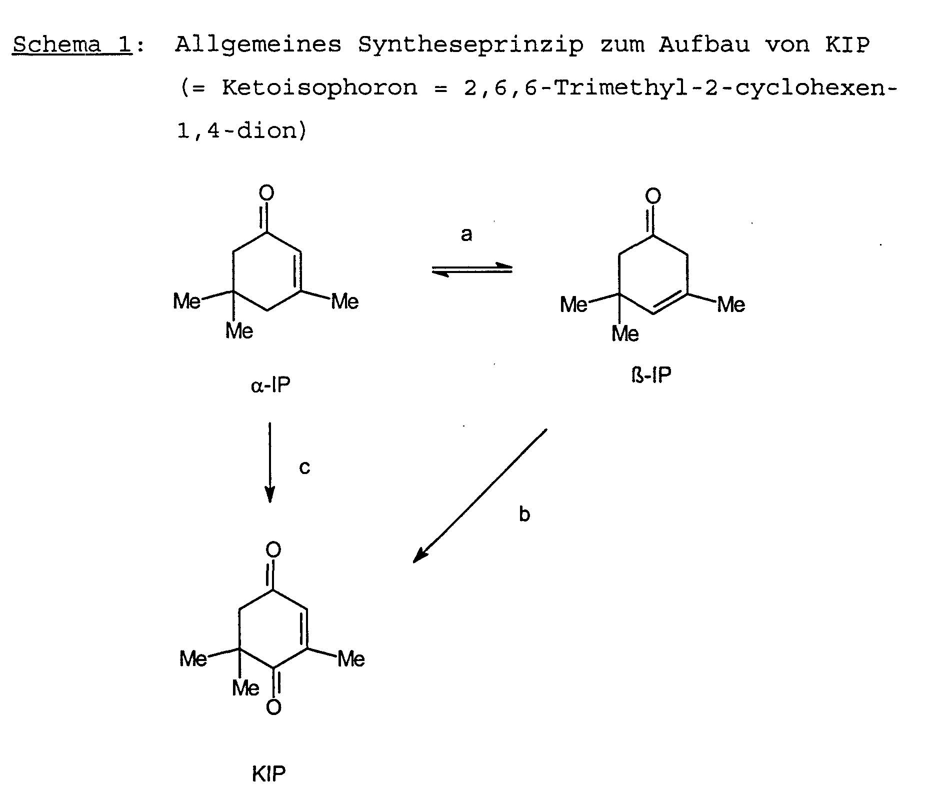
a = Umisomerisierung von α-IP zu β-IP
b = Oxidation von β-IP zu KIP
c = Direktoxidation α-IP zu KIP

Im Laufe der Zeit sind zahlreiche Verfahren zur Isomerisierung von α-IP beschrieben worden, die jedoch erhebliche Nachteile aufweisen. Gesichtspunkte wie hoher Chemikalienverbrauch, schlechte Raum-/Zeitausbeute und Schwierigkeiten bei der Aufarbeitung haben bisher eine praktische Verfahrensumsetzung im größeren Maßstab verhindert.

Bei den Herstellungsverfahren für β-IP aus α-IP kann man zwischen Gasphasen- und Flüssigphasen-Reaktionen unterscheiden.

Grundsätzlich sind vier parallele Reaktionen von α-Isophoron in der Gasphase möglich, die miteinander in Konkurrenz stehen und abhängig vom gewählten Temperaturbereich bzw. der Oberfächenbeschaffenheit des eingesetzten Katalysators in unterschiedlichem Ausmaß zum Zuge kommen.

Isophoron kann auf folgende Weise am Kontakt in der Gasphase reagieren:
a.) Isomerisierung zu β-Isophoron
b.) Reduktion zu Trimethylcyclohexadienen (hierzu benötigter Wasserstoff wird durch IP-Zersetzung geliefert, die von Verkokungserscheinungen begleitet wird)
c.) β-Eliminierung von Methan zu 3,5-Xylenol
d.) Mesitylen-Entstehung

Im folgenden Schema 2 sind die am heterogenen Kontakt in der Gasphase katalysierten Reaktionen von α-IP dargestellt:

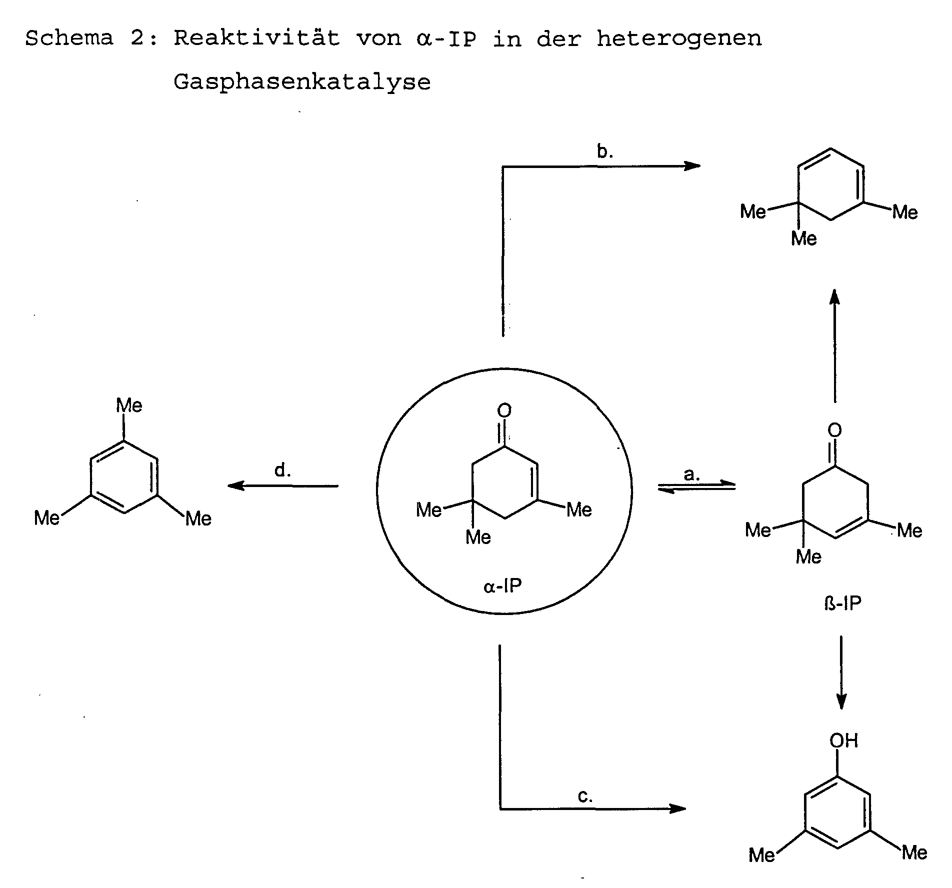

Die EP 0 488 045 B1 offenbart ein Isomerisierungsverfahren in der Gasphase (300 - 450 °C) über einem heterogenen Katalysator. Als Katalysatoren werden Oxide und Mischoxide von Mg (Gr. IIa), Al (IIIa), Si (IVa) und Ni (VIII) verwendet, die per se aktiv sind oder auf einem γ-Aluminium-Oxid-Träger aufgebracht werden (spezifische Oberfläche 1 - 50 m²/g). Pro Liter Katalysator werden 1 - 10 kg α-IP eingesetzt, die Konzentration der intermediär erhaltenen Lösung beträgt ~ 9 % β-IP, die Endkonzentration nach Destillation im Vakuum 97 % β-IP. Die Granulierung von NiO erfolgt mit 1 % Luviskol K90 (-Polyvinylpyrrolidon). Dieses Ergebnis entspricht bezogen auf die eingesetzte Katalysator-menge und Zeit einer Ausbeute von Y = 0,308 kg
β-IP pro Liter Katalysator und Stunde. Nachteilig hieran ist, daß nur ein 9 % β-IP-Gemisch pro Stunde anfällt. Bezogen auf das eingesetzte Eduktvolumen beträgt die Raum-Zeit Ausbeute Y_{R-Z} = 0,09 1_{β-IP}/h/l_{Lösung} (Bsp.1)

Zudem ist die Abnahmegeschwindigkeit gering, was das Verfahren im industriellen Maßstab wenig attraktiv macht.

Bei L. F. Korzhova, Y. V. Churkin und K. M. Vaisberg, Petrol. Chem Vol. 31, 1991, 678 wird die Umsetzung von α-IP bei 300 - 800 °C in Gegenwart heterogener Katalysatoren beschrieben. Als katalytische Systeme werden γ-Aluminiumoxid, Magnesiumoxid und Quarz berücksichtigt. Das Produktspektrum wird in Abhängigkeit von Temperatur und Katalysator betrachtet. Verglichen miteinander werden die Bildung von β-IP, Trimethylcyclohexadien, 3,5-Xylenol, und Mesitylen (siehe Schema 2: Weg a., b., c.; d.). So ergibt die thermische Umsetzung von α-IP bei über 550 °C an einer wenig ausgebildeten katalytischen Oberfläche (Quarz) ein Gemisch der Zusammensetzung c >> a >> d und b = 0. Die Umsetzung am MgO-Kontakt bei 400 °C zeigt ein ähnliches Produktbild bei deutlich niedrigerer Temperatur, nämlich c >> a > d > b. In Gegenwart eines Aluminiumoxidkatalysators mit ausgeprägt basisch-saurer Oberflächenstruktur erfolgt die Umsetzung bei 300 °C mit deutlicher Bevorzugung der Cyclohexadien Produkte, nämlich b >> c > d.

Insgesamt ist davon auszugehen, daß eine katalytische Gasphasenisomerisierung durchaus auf mehrfache Weise nachteilig ist: Allgemein läßt sich sagen, daß diese Verfahren sich nachteilig darstellen, weil entweder die Produktbildung von einem erheblichen Nebenproduktanfall begleitet wird, oder die Raum-Zeit-Ausbeute (absolute β-IP-Entstehung/hkg_{Kat}) zu gering ist.

Auch mit der Isomerisierung in der Flüssigphase beschäftigen sich eine Reihe von Druckschriften. Der nähere Stand der Technik wird durch folgende Schriften repräsentiert:
- D1 =: A. Heymes et al., Recherches 1971, 18, 104
- D2 =: FR-A-1 446 246
- D3 =: DE-OS-24 57 157
- D4 =: US-A-4,005,145
- D5 =: EP-A-0 312 735
- D6 =: JP 87-33019 entspr. HEI-1-175954 v. 12.07.1989

D1 offenbart die Isomerisierung von α-IP zu β-IP mit stöchiometrischen Mengen MeMgX (X = Halogen-) Grignard Verbindung. Man erhält unter Methanfreisetzung in Gegenwart katalytischer Mengen FeCl₃ 73 % β-IP. Mechanistische Vorstellungen gehen davon aus, daß die Grignardverbindung als Base reagiert und nicht als Überträger eines Carbanions fungiert. Überschüssiges Mg führt zur Entstehung von Dimerengemischen, die aus einer reduktiven metallischen Dimerisierung hervorgehen. Die Umsetzung von α-Isophoron mit molaren Mengen Methylmagnesiumjodid in Gegenwart katalytischer Mengen FeCl₃, anschließende Hydrolyse und destillative Aufarbeitung ist jedoch ebenso kompliziert wie chemikalienaufwendig.
D2 betrifft die Umisomerisierung von α-IP zu β-IP in Gegenwart katalytischer Mengen p-Toluolsulfonsäure und allgemein aromatischer Sulfonsäuren, insbesondere Anilinsulfonsäure. Die Einsatzmenge des Katalysators beträgt bezogen auf eingesetztes α-IP 0,1 - 0,2 %. Ein niedriger Umwandlungsgrad und ein hoher Nebenproduktanfall verhindern jedoch eine industrielle Anwendung des Verfahrens der D2.

Nach der D3 erfolgt die Darstellung von β-IP durch mehrstündiges Kochen von α-IP in Triäthanolamin, Fraktionierung, Waschen des Destillates mit Weinsäure und Kochsalzlösung. Auch hier ist der Chemikalienverbrauch erheblich.

In der D4 werden als Katalysator Säuren eingesetzt mit einem pK = 2 - 5 und einem höheren Siedepunkt als β-IP (Sdpkt. β-IP = 186 °C/760 mm Hg). Gemäß Patentanspruch sind in der flüssigen Phase explizit geschützt:

Aliphatische und aromatische Aminosäuren, Adipinsäure, p-Methyl-Benzoesäure, 4-Nitro-m-Methyl-Benzoesäure, 4-Hydroxy-Benzoesäure, 3,4,5-Trimethoxybenzoesäure, Vanillinsäure, 4-Trifluormethyl-Benzoesäure, 3-Hydroxy-4-Nitrobenzoesäure und Cyclohexancarbonsäure und Derivate. Der Katalysatoreinsatz beträgt 0,1 - 20 Mol %. Die Ausbeute an β-IP (bezogen auf eingesetztes α-IP) beträgt 74,5 %. Dies entspricht unter den gegebenen Bedingungen umgerechnet auf die eingesetzte Katalysatormenge und Zeit einer Ausbeute von Y = 0,218 Liter β-IP pro Kilogramm Katalysator und Stunde.

Die homogen-katalytische Isomerisierung von α-IP→β-IP mit wenig dissoziierten Säuren stellt eine Verbesserung bezüglich des Chemikalienverbrauchs dar, wobei β-IP kontinuierlich aus dem Gleichgewicht entfernt wird. Mit einer so geringen Abnahmegeschwindigkeit wie 11 ml/h β-IP bei einer Einsatzmenge von etwa 0,5 kg α-IP ist die Raum-Zeit-Ausbeute und die β-IP-Entstehung mit Y = 0,24 kg β-IP/kg_{Kat}•h zu niedrig, um technische Anwendung zu finden.

Nach einem ähnlichen Prinzip geht man in D5 vor. Als π-Bindungsverschiebungskatalysatoren finden Acetylacetonate von Übergangsmetallen Verwendung. Auch Al(acac) zeigt katalytische Aktivität. Der Einsatz des Katalysators erfolgt in 0,01 - 10 wt %. Patentiert sind Metallkatalysatoren der Gruppen IVb (Ti/Zr/Hf), Vb (V/Nb/Ta), VIb (Cr, Mo, W), VIIb (Mn/ Tc/Re) der gesamten Gruppe VIII und Aluminium. Das primär anfallende Destillat hat einen β-IP-Gehalt von 94 %, eine weitere Vigreuxdestillation reichert den β-IP-Gehalt auf 99 % an. Dieses Ergebnis entspricht bezogen auf Katalysatoreinsatzmenge und Zeit einer Ausbeute von Y = 9,4 Liter β-IP pro Kilogramm Katalysator und Stunde.Bezogen auf die eingesetzte Eduktlösung entspricht dies einer Ausbeute von Y_{R-Z} = 0,0376 l_{β-IP}/h/l_{Lösung}.

Abgesehen davon, daß die Raum-Zeit-Ausbeute gering und der Nebenproduktanfall beträchtlich ist, lassen sich bei dem eingesetzten homogenen Katalysatorsystem Katalysator und Destillationsrückstand nicht ohne weiteres trennen. Daher ist ein Verwerfen von Zeit zu Zeit unerläßlich, da die Temperatur im Destillationssumpf sonst zu sehr ansteigen würde. Auch so ist schon ein "Nachziehen" der Temperatur erforderlich.

Gemäß D6 erfolgt die Isomerisierung in der Flüssigphase bei Temperaturen um 200 °C. Als Katalysator finden Kieselgel mit oder ohne Zusatz von alkylsubstituierten Imidazolinen der nachfolgenden Formel Verwendung. 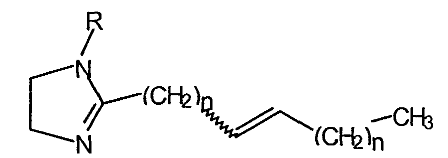 R = ß-Hydroxyethylgruppe, ß-Hydroxypropylgruppe oder γ-Hydroxypropylgruppe, m = ganze Zahl zwischen 5 und 7, n = ganze Zahl zwischen 0 und 12

Typische Experimentbedingungen: 300 g α-IP und 25,7 g SiO₂ werden 52 h in Gegenwart von Edelstahl destilliert, es resultieren 230 g β-IP (= 76,6 % Ausbeute) mit 99,9 % Reinheit. Dieses Ergebnis entspricht bezogen auf Katalysatoreinsatzmenge und Zeit einer Ausbeute von Y = 0,174 Liter β-IP pro Liter Katalysator und Stunde.

Die Präparation der organischen Basen ist jedoch teuer und die Raum-Zeit-Ausbeute des Verfahrens gering; mit einem charakteristischen Wert von Y = 0,174 Liter β-IP/l Kat•h ist auch dieses Verfahren nicht in einen technischen Maßstab umzusetzen.Bezogen auf das Volumen der eingesetzten Eduktlösung beträgt die Ausbeute Y_{R-Z} = 0,0149 l_{ß-} _{IP}/h/l_{Lösung}.

Die beschriebene Verfahrensweise ist zudem ungünstig, die absolute β-IP-Entstehung gering. Besonders nachteilig ist die batchweise Durchführung und die Durchführung von Isomerisierung und Reindestillation des β-IP in einem Schritt. Durch die hohe Reaktionstemperatur in der Destillationsapparatur tritt nachweislich im erheblichen Maße die Rückisomerisierung von β-IP zu α-IP ein.

Angesichts des hierin zitierten und diskutierten Standes der Technik war es eine Aufgabe der Erfindung, die oben erwähnten Nachteile der bisherigen Methoden zu vermeiden und ein Verfahren anzubieten, nach dem auf technisch vorteilhafte Weise 3,5,5-Trimethylcyclohexa-3-en-1-on aus seinem Isomeren 3,5,5-Trimethylcyclohexa-2-en-1-on hergestellt werden kann. Insbesondere war das Auffinden eines heterogen katalytischen Verfahrens in der Flüssigphase ein besonderes Ziel der Erfindung.

Gelöst werden diese sowie weitere nicht einzeln angegebene Aufgaben bei einem Verfahren der eingangs genannten Gattung durch die im kennzeichnenden Teil des Anspruches 1 angegebenen Maßnahmen.

Dadurch, daß als Katalysator ein Oxid oder Mischoxid eines Elements der Gruppen IIₐ, VIII, I_{b}, IIIₐ und Vₐ des Periodensystems oder ZnO, TiO₂, ZrO₂ oder MoO₃ verwendet wird, und die Isomerisierung ohne Zusatz einer organischen Base ausgeführt wird, gelingt es in nicht ohne weiteres vorhersehbarer Weise, die Raum-Zeit-Ausbeute bei der Herstellung von β-Isophoron durch Isomerisierung von α-Isophoron auf ein für eine industrielle Anwendung geeignetes Niveau zu heben und gleichzeitig einen aus dem Stand der Technik vorbekannten Prozeß deutlich zu vereinfachen.

Das erfindungsgemäße Verfahren ermöglicht einen hohen Umsatz im Bereich von ca. 9 kg α-Isophoron pro kg eingesetztem Katalysator und pro Stunde und übertrifft dadurch die bislang im Stand der Technik bekannten Verfahren. Weiterhin werden erfindungsgemäß sowohl der Anfall von Nebenprodukten verringert als auch die Raum-Zeit-Ausbeute bezogen auf das Volumen der eingesetzten Eduktlösung verbessert. Insgesamt gesehen, ist die Benutzung des erfindungsgemäßen heterogenen Katalysators in jedem Falle vorteilhaft.

In der vorliegenden Erfindung wird ein Verfahren eingesetzt, in dem α-Isophoron zu seinem Isomeren β-Isophoron umgesetzt wird, wobei ein heterogener Katalysator in der Flüssigphase verwendet wird.

Besonders vorteilhaft ist eine Verfahrensweise, bei der Reaktion und Produktisolierung nicht in derselben Apparatur stattfinden. Dadurch, daß man in einer Isomerisierungseinheit zunächst ein Gemisch von α-IP und β-IP erzeugt, und dann die Reindestillation im Vakuum durchführt, kann die Raum-Zeit-Ausbeute deutlich gesteigert werden, da bei einer Aufkonzentration von β-IP bei Normaldruck und der Siedetemperatur von 186 ° C teilweise Rückisomerisierung eintritt, die durch schnelles Entfernen der Reaktionsmischung aus dem Reaktionsraum verhindert wird.

Als heterogene Katalysatoren im Sinne der Erfindung werden Oxide oder Mischoxide eines Elements der Gruppen IIₐ, VIII, I_{b}, IIIₐ und Vₐ des Periodensystems der Elemente oder auch unter Versuchsbedingungen unlösliche Salze der genannten Elemente, wie insbesondere Carbonate oder Halogenide oder ZnO, TiO₂, ZrO₂ oder MoO₃ eingesetzt. Die Gruppeneinteilung der Haupt- und Nebengruppen des Periodensystems der Elemente erfolgt nach der Bezeichnung gemäß IUPAC, Pure and Appl. Chem., 66, 2423-2444,1994. So gehören zur Gruppe IIₐ die Metalle Be, Mg, Ca, Sr, Ba und Ra. Zur Gruppe VIII die Metalle Fe, Co, Ni, Ru, Rh, Pd, Os, Ir und Pt. Zu den Gruppen I_{b}, IIIₐ und Vₐ die Elemente Cu, Ag, Au, B, Al, Ga, In, Tl, N, P, As, Sb und Bi.

Zu den erfindungsgemäß als heterogene Katalysatoren einsetzbaren Verbindungen gehören die Oxide oder Mischoxide der vorgenannten Elemente. Hierbei wird unter Mischoxid eine Verbindung verstanden, in der Sauerstoff mit mehr als einem der aufgezählten Elemente eine Verbindung eingeht.

Zu den im Rahmen der Erfindung einsetzbaren Oxiden gehören BeO, MgO, CaO, SrO, BaO, TiO₂, ZrO₂, MoO₃, Fe₃O₄, Fe₂O₃, CoO, Co₃O₄, NiO, PdO₂, PtO₂, ZnO, Al₂O₃.

Zu den im Rahmen der Erfindung einsetzbaren Mischoxiden gehören neben Mischverbindungen oben aufgezählter Oxide unter anderem auch Al₂O₃/SiO₂ und Zeolithe verschiedener Module, z.B.H-ZSM-5.

Von den o. g. Oxiden oder Mischoxiden sind insbesondere diejenigen bevorzugt, welche ein Element der Gruppen IIₐ oder VIII des Periodensystems enthalten.

Ganz besonders bevorzugt werden im Rahmen der Erfindung Oxide oder Mischoxide des Calciums und/oder Magnesiums verwendet.

In einer weiteren Ausgestaltung des erfindungsgemäßen Verfahrens ist es bevorzugt, daß ein Oxid oder Mischoxid des Kobalt und/oder Nickel verwendet wird.

Ganz besonders bevorzugte Oxide sind u. a. Co₃O₄ sowie MgO und CaO.

Ein weiterhin besonders bevorzugter Katalysator ist γ -Al₂O₃. Besonders geeignet sind Cobalt- und Nickelcarbonate, gegebenenfalls in ihrer Hydratform.

Neben der Verwendung von Oxiden oder Mischoxiden als erfindungsgemäße heterogene Katalysatoren zur Isomerisierung von α-Isophoron zu β-Isophoron können mit gutem Erfolg auch mit elementaren Metallen dotierte Oxide und Mischoxide der Gruppen IIₐ, VIII, I_{b}, IIIₐ und Vₐ des Periodensystems der Elemente eingesetzt werden. Zur Dotierung können Elemente, insbesondere Metalle, aus denselben Gruppen des Periodensystems verwendet werden. Zu den vorzugsweise einzusetzenden Dotierungsmetallen gehören u. a. die Metalle der Gruppen VIII sowie I_{b}. In besonderer Abwandlung kennzeichnet sich das erfindungsgemäße Verfahre dadurch, daß ein Katalysator eingesetzt wird, der mit eine Metall aus der Gruppe VIII des Periodensystems dotiert ist Innerhalb der Gruppe VIII wiederum sind die Metalle Kobalt und/oder Nickel als Dotierungsmetalle besonders günstig.

Die Menge des zur Dotierung eingesetzten Metalles ist an sich nicht besonders kritisch und kann daher über einen weiten Bereich variiert werden. Bevorzugt ist es, daß das Dotierungsmetall in einer Menge von 0,1 bis 50 Gew.-% (wt/wt) bezogen auf das Oxid oder Mischoxid eingesetzt wird. Ein besonders günstiger Katalysator wird erhalten, wenn ein mit Nickel und/oder Kobalt dotiertes γ-Al₂O₃ oder Co₃O₄ eingesetzt wird.

Ferner kann der Katalysator bzw. auch der mit einem Metall dotierte Katalysator in reiner Form vorliegen oder auf einem Trägermaterial fixiert oder mit dem Träger gemischt sein, wobei das Trägermaterial einer der beschriebenen Katalysatoren sein kann. Weitere Trägermaterialien sind dem Fachmann bekannt. Hierzu gehören Träger wie α-Al₂O₃, γ-Al₂O₃, SiO₂/Al₂O₃ verschiedener Module, Aluminiumsalze wie etwa Aluminiumsilikate und Aluminiumphosphate, Aktivkohle etc.

Auch die Menge des zur Isomerisierung einzusetzenden Katalysators kann grundsätzlich über einen größeren Bereich variiert werden. Bevorzugt ist es hierbei, daß der Katalysator in einem Verhältnis von zwischen 0,01 und 30 Gew.-% (wt/wt) bezogen auf α-Isophoron eingesetzt wird. In einer besonders bevorzugten Ausführungsvariante kennzeichnet sich das Verfahren der Erfindung dadurch, daß der Katalysator in einem Verhältnis von zwischen 0,2 und 10 Gew.-% (wt/wt) bezogen auf α-Isophoron eingesetzt wird.

In noch-einer weiteren besonders bevorzugten Ausführungsform liegt das Verhältnis von Katalysator zu α -Isophoron im Bereich zwischen 0,5 und 5 Gew.-% (wt/wt).

Das Verfahren gemäß Erfindung wird über einen Temperaturbereich zwischen 100 und 300 °C ausgeführt. Bevorzugt ist der Temperaturbereich zwischen 150 und 260 ° C.

Der Zusatz eines Verdünnungs- oder Lösungsmittels ist zwar möglich, aber nicht erforderlich.

Vorzugsweise wird die Reaktion bei einem Druck von 1x10⁴ Pa bis 3x10⁵ Pa Überdruck durchgeführt. Ganz besonders günstige Isomerisierungsparameter sind ~10³ Pa bis Normaldruck (etwa 1x10⁵)Pa in Kombination mit der Siedetemperatur des α-Isophorons.

Das erfindungsgemäße Verfahren läßt sich hervorragend kontinuierlich betreiben. In einer bevorzugten Ausführungsform trennt man Isomerisierung und Reindestillation voneinander.Die das Isomerisat enthaltende Flüssigphase wird nach deren Abtrennung zur Trennung von α-Isophoron und β-Isophoron bei einem Druck von 100 bis 3x10⁴ Pa destilliert. Das Reaktionsgemisch wird in einer Menge von 5 bis 95 wt %h ab- gezogen.

Die Destillation findet dann bei Temperaturen statt, bei denen die thermische bedingte Rückisomerisierung weitgehend ausgeschlossen ist.

Dabei erweist es sich als vorteilhaft, das Sumpfprodukt der Destillation in die Isomerisierungsstufe zu recyclieren.

Die Zufuhr von α-Isophoron beträgt 2,3 - 70 1 α-IP/h•kg Kat, wobei am Kopf der Isomerisierungseinheit ein 5- - 60-%-Gemisch β-IP/ α-IP abgenommen wird. Dieses Gemisch wird anschließend einer Vakuumdestillation unterworfen, wobei ein β-IP-Produkt mit einer Reinheit > 97 % anfällt. Das Sumpfprodukt der Kolonne wird ohne Reinigung auf die Isomerisierungseinheit zurückgefahren. Auf diese Weise ist es möglich, umgerechnet 9,182 kg β-IP/kg Kat●·h zu erzeugen. Dieses Ergebnis übertrifft das der bislang beschriebenen Verfahren. Auch hinsichtlich des Nebenproduktanfalls (im Vergleich z.b. mit D1, wo stöchiometrische Mengen Grignard-verbindung verbraucht werden)und der auf das Volumen der Eduktlösung bezogene Raum-Zeit-Ausbeute ist das neue Verfahren dem Stand der Technik deutlich überlegen. Die Benutzung eines heterogenen Katalysators bringt zusätzlich erhebliche Vorteile bei der Abtrennung der Hochsieder vom Katalysatormaterial. Hier zeigen bekannte Verfahren(z. B. EP 312 735)deutliche Nachteile, die beim erfindungsgemäßen Verfahren nicht auftreten. Eine Filtration und Nachbehandlung bzw. Regenerierung des Katalysators mit wenig Lösungsmittel (z. B. α-IP selbst) ist vollkommen ausreichend.

### Beispiel 1:

Es wird käufliches Co₃O₄ der Firma Merck (Co(II/III)oxid) verwendet. Die Katalysatoreinsatzform ist in diesem Fall pulverförmig, jedoch ist auch eine granulierte Form katalytisch aktiv. Eine Vorbehandlung des Katalysators ist nicht notwendig. Die Apparatur zur Durchführung der Isomerisierung besteht aus einem Umlauferhitzer, der mit zwei elektrisch versorgten Stabeinsätzen beheizt wird. Man legt 700 ml technisches α-Isophoron vor (Firma Atochem > 98 %) und gibt 25 g Co₃O₄ hinzu. Über dem Umlauferhitzer sitzt eine Destillationskolonne mit 1,2 m Länge und 25 mm Innendurchmesser, die mit V4A Raschigringen von 4 mm Ø gefüllt ist. Man erhitzt die Suspension bei Normaldruck bis zur Siedetemperatur und stimmt die Mengen des über eine Telabpumpe zugeförderten α-IP und des abgenommenen Destillats aufeinander ab. In Abhängigkeit von der Abnahmegeschwindigkeit stellt sich folgender β-IP-Gehalt im Primärdestillat ein.

| **Abnahmegeschwindigkeit** | **20 ml/h** | **40 ml/h** | **80 ml/h** | **120 ml/h** | **160 ml/h** | **260 ml/h** |
|---|---|---|---|---|---|---|
| Gehalt β-IP [ml] | 47,3 | 44,4 | 38,9 | 33,6 | 26,8 | 19,0 |
| Entstehung β-IP/h [ml/h] | 9,4 | 17,7 | 31,1 | 40,4 | 43,5 | 49,5 |

Die Sumpftemperatur der Isomerisierung bliebt während der Reaktionsdauer konstant bei 216-217 °C. Das anfallende Primärdestillat wird auf eine Destillationskolonne gefördert, die bei einem Unterdruck von 5 mbar - 100 mbar arbeitet. Das bei 12 mbar anfallende Kopfprodukt hat einen Siedepunkt von 55 - 58 °C und besteht zu > 97 % aus β-Isophoron. Bei der beschriebenen Dimensionierung des Versuchs entstehen pro Stunde 50 g β-Isophoron. Das als Sumpfprodukt nicht umge-setzte α-Isophoron wird mit einem Rest-β-Gehalt < 3 % auf die Isomerisierungseinheit zurückgefahren. Die Selektivität bezogen auf den Umsatz beträgt > 98 %. Die Ausbeute bezogen auf die eingesetzte Katalysatormenge beträgt Y=1,98 l_{β-IP}/h/kgₖₐₜ. .Die Raum-Zeit Ausbeute bezogen auf das Volumen der zu isomerisierenden Lösung beträgt Y_{R-Z} = 0,0707 I_{ß-IP}/h/l_{Lösung}.

### Beispiel 2

25 g eines Magnesiumoxidkatalysators werden in die bereits beschriebene Apparatur eingefüllt. Bei gleicher kontinuierlicher Fahrweise (siehe Beispiel 1) wird am Kopf der Isomerisierungseinheit je nach Abnahmegeschwindigkeit ein Primärdestillat mit folgender Zusammensetzung abgenommen:

| **Abnahmegeschwindigkeit** | **40 ml/h** | **120 ml/h** | **240 ml/h** |
|---|---|---|---|
| Gehalt β-IP [ml] | 46,0 | 34,8 | 21,5 |
| Entstehung β-IP/h [ml/h] | 18,4 | 41,8 | 51,6 |

Durch weitere Erhöhung der Abnahmegeschwindigkeit kann die absolut entstehende β-IP-Menge optimiert werden.Die Sumpftemperatur der Isomerisierung beträgt über die Dauer der Reaktion konstant 216-217 °C. Die Ausbeute bezogen auf die eingesetzte Katalysatormenge beträgt Y=2,064 l_{βIP}/h/kg_{Kat.}.Die Raum-Zeit Ausbeute bezogen auf die eingesetzte Eduktlösung beträgt Y_{R-Z} = 0,0737 l_{βIP}/h/l_{Lösung}.

### Beispiel 3:

In der beschriebenen Apparatur (gleiche Dimensionierung der Isomerisierung wie Beispiel 1) werden 1.160 1 α-IP an 4,4 g Co₃O₄-Katalysator umgesetzt (Kobaltschwarz Co₃O₄; MW = 240,8 g/mol; 4,4 g = 18,3 mmol) (IP = 138,21 g/mol; 1.160 ml = 8.393 mmol). In Abhängigkeit der Abnahmegeschwindigkeit erhält man Primärdestillate folgenden β-IP-Gehalts:

| **Abnahmegeschwindigkeit** | **20 ml/h** | **40 ml/h** | **80 ml/h** | **160 ml/h** | **240 ml/h** | **280 ml/h** |
|---|---|---|---|---|---|---|
| Gehalt β-IP [ml] | 52,6 | 47,3 | 40,4 | 25,9 | 16,6 | 14,7 |
| Entstehung β-IP/h [ml/h] | 10,5 | 18,9 | 32,2 | 41,4 | 39,8 | 41,2 |

Die Sumpftemperatur der Isomerisierungseinheit beträgt konstant 216-217 °C. Die Ausbeute bezogen auf die eingesetzte Katalysatormenge beträgt Y=9,363 l_{βIP}/h/kg_{Kat.} .Die Raum-Zeit Ausbeute bezogen auf die eingesetzte Eduktlösung beträgt Y_{R-Z} = 0,0588 l_{βIP}/h/l_{Lösung}.

### Beispiel 4:

Anstelle des Kobaltoxid-Katalysators aus Beispiel 3 wird α-Aluminiumoxid (Firma Hoffmann La Roche A2) in der Isomerisierungseinheit eingesetzt. Die Reaktion wird analog Beispiel 1 durchgeführt. Man erhält bei kontinuierlicher Abnahme Gemische α-IP/β-IP der folgenden Zusammensetzung:

| **Abnahmegeschwindigkeit** | **20 ml/h** | **40 ml/h** | **95 ml/h** | **160 ml/h** | **180 ml/h** |
|---|---|---|---|---|---|
| Gehalt β-IP [ml] | 58,4 | 34,5 | 17,6 | 12,2 | 9,7 |
| Entstehung β-IP/h [ml/h] | 11,7 | 13,8 | 16,7 | 19,5 | 17,5 |

Die Isomerisierung wird bei einer konstanten Sumpftemperatur von 216-217 °C durchgeführt. Die Ausbeute bezogen auf die eingesetzte Katalysatormenge beträgt Y=0,78 l_{β-IP}/h/kg_{Kat.} .Die Raum-Zeit Ausbeute bezogen auf die eingesetzte Eduktlösung beträgt Y_{R-Z} = 0,0278 l_{βIP}/h/l_{Lösung}.

### Beispiel 5:

Als Katalysator wird Co₃O₄ (Firma Merck) ohne Vorbehandlung eingesetzt. Die Dimensionierung der Apparatur entspricht den vorherigen Beispielen, die stöchiometrischen Verhältnisse Katalysator / α-IP entsprechen den Bedingungen von Beispiel 1. Man variiert Druck und Temperatur der Isomerisierungseinheit und untersucht bei konstanter Abnahmegeschwindigkeit von 120 ml/h den β-IP-Gehalt des am Kopf der Kolonne anfallenden Primärdestillats. Die zu den entsprechenden Drücken zugeordneten Isomerisierungstemperaturen sind der Tabelle zu entnehmen.

| **Temperatur Sumpf [°C]** | **Druck [mbar]** | **Gehalt β-IP [FL.%]** |
|---|---|---|
| 216 | 1000 | 33,8 |
| 203 | 770 | 25,8 |
| 192 | 580 | 18,6 |
| 178 | 390 | 14,6 |

### Beispiel 6:

Man betreibt die in den Beispielen 1 - 5 beschriebenen Apparate halbkontinuierlich (nicht umgesetztes α-IP wird nicht erneut mit dem Katalysator in Kontakt gebracht) und befüllt die Isomerisierungseinheit mit 25 g Co₃O₄ (Firma Merck). Dann dosiert man mit einer Telab-Laborpumpe kontinuierlich insgesamt 11 l technisches α-IP zu, wobei ein etwa 20-Fl.%-ß-IP/α-IP-Gemisch als Primärdestillat anfällt. Die Sumpftemperatur der Isomerisierungseinheit bleibt während der Reaktionszeit bei konstant 216-217 °C.

Die Abnahmegeschwindigkeit beträgt ~ 250 ml/h, was einer β-IP-Entstehung von 50 [ml β-IP/h] entspricht. Als Sumpfprodukt verbleiben 905 g eines dünnen Öls, das zu 117 g (12,9 %) aus Überkondensaten besteht und zu 87,1 % aus wiedergewinnbarem α-IP. Damit beträgt der Nebenproduktanfall bezogen auf umgesetztes α-IP 5,3 %.

### Beispiel 7:

Man betreibt die in den Beispielen 1 - 5 beschriebenen Apparate kontinuierlich. Die Isomerisierungseinheit ist über eine Telabpumpe mit der Destillationskolonne verbunden. Das im Sumpf der Destillationseinheit anfallende α-IP wird über einen Überlaufbehälter abgenommen und wieder der Isomerisierung zugeführt. Am Kopf der Destillationskolonne wird ein β-Isophoron mit einer Reinheit > 97 % abgenommen. Auf diese Weise werden 3,7 1 α-IP (Atochem: > 98 % GC) umgesetzt. Als Katalysator werden 25 g Kobaltschwarz (Fa. Merck) eingesetzt, die Abnahmegeschwindigkeit beträgt 240 - 250 ml/h, die Isomerisierungstemperatur beträgt 216-217 °C. Das Primärgemisch hat einen β-IP-Gehalt von 20 - 22 %. Während der Reaktionsdauer zeigt der Katalysator keine Alterung und kann am Schluß der Reaktion durch Filtration fast vollständig zurückgewonnen werden (23,3 g Co₃O₄). Nach Abschluß der Reaktion verbleiben im Umlaufverdampfer 555 g α-IP und 60 g Hochsieder, die problemlos destillativ getrennt werden können. Als Destillat erhält man 3,07 kg β-IP (Reinheit ~ 98 %). Die Ausbeute bezogen auf den Umsatz beträgt also 97,6 %, der Nebenproduktanfall beträgt 1,9 %. Der Rest besteht aus Wasser, das durch α-IP-Dimerisierung (= Kondensation) entsteht, bzw. durch das technische Edukt in die Reaktion gelangt.

### Beispiel 8

In einen 2-l-Dreihalskolben mit KPG-Rührer und aufgesetzer 120-cm-Vigreux-Kolonne werden 50 g CaO als Katalysator gegeben und 1,5 l α-Isophoron vorgelegt. Man erniedrigt den Druck der Apparatur auf 350 mbar, wobei die Flüssigkeit bei einer Innentemperatur von 175 - 180 C zu sieden beginnt.

Der Dreihalskolben ist zusätzlich mit einem Tropfrichter ausgestattet, der eine kontinuierliche Zugabe von α-IP erlaubt. Die Zugabe von Frisch-α-IP entspricht der Menge an am Kopf der Vigreuxkolonne abgenommener α-IP/β-IP-Mischung. Man nimmt kontinuierlich 200 ml Isomerengemisch ab, deren β-Gehalt etwa 21 - 22 Gew.-% beträgt. Die entstehende Mischung wird im Vakuum destilliert, das im Sumpf der Reindestillation anfallende α-IP wird wieder zum Katalysator zudosiert. Am Kopf der Reindestillation kann β-IP-Produkt mit einer Reinheit >98 % abgenommen werden. Mit dieser Verfahrensweise werden 3 kg α-IP umgesetzt und man erhält 2.850 g eines >98 %-β-IP-Produktes. Die Selektivität, bezogen auf umgesetztes α-IP, beträgt >95 %. Der Katalysator ist nach Regenerierung durch Filtration und Waschen mit α-IP noch aktiv und kann für einen weitere Zyklus eingesetzt werden. Die Ausbeute bezogen auf die eingesetzte Katalysatormenge beträgt Y=0,88 l_{β-IP}/h/kg_{Kat.} .Die Raum-Zeit Ausbeute bezogen auf die eingesetzte Eduktlösung beträgt Y_{R-Z} = 0,0293 l_{β} _{IP}/h/l_{Lösung}.

### Beispiel 10

Man benutzt die gleiche Apparatur wie in Beispiel 9 beschrieben und verwendet 5 wt% CoCO₃ (=Kobaltcarbonat, Fa. AMG Kokkola) als Katalysator. Bei einer Abnahmegeschwindigkeit von 25 vol% der eingesetzten α-IP Mischung beträgt die β-IP Bildungsrate 67 g/h/l. Durch Quantifizierung des entstehenden Hochsiederanteils wird eine Selektivität von S = 98 % bestimmt

## Patentansprüche

1. Verfahren zur Herstellung von 3,5,5-Trimethylcyclohexa-3-en-1-on (β-Isophoron) durch Isomerisierung von 3,5,5-Trimethylcyclohexa-2-en-1-on (α-Isophoron) in der Flüssigphase in Gegenwart eines heterogenen Katalysators,
**dadurch gekennzeichnet**,
daß als Katalysator ein Oxid oder Mischoxid eines Elements der Gruppen IIₐ, VIII, I_{b}, IIIₐ und Vₐ des Periodensystems oder unter Versuchsbedingungen unlösliche Salze der genannten Elemente ZnO, TiO₂, ZrO₂ oder moO₃ verwendet wird, und die Isomerisierung ohne Zusatz einer organischen Base ausgeführt wird.

2. Verfahren nach Anspruch 1,
**dadurch gekennzeichnet**,
daß ein Oxid oder Mischoxid eines Elements der Gruppen IIₐ und VIII des Periodensystems verwendet wird.

3. Verfahren nach Anspruch 2,
**dadurch gekennzeichnet**,
daß ein Oxid oder Mischoxid des Ca und/oder Mg verwendet wird.

4. Verfahren nach Anspruch 2,
**dadurch gekennzeichnet**,
daß ein Oxid oder Mischoxid des Co und/oder Ni verwendet wird.

5. Verfahren nach Anspruch 4,
**dadurch gekennzeichnet**,
daß Co₃o₄ eingesetzt wird.

6. Verfahren nach einem oder mehreren der vorhergehenden Ansprüche,
**dadurch gekennzeichnet**,
daß ein Katalysator eingesetzt wird, der mit einem Metall aus der Gruppe VIII des Periodensystems dotiert ist.

7. Verfahren nach Anspruch 6,
**dadurch gekennzeichnet**,
daß ein mit Ni und/oder Co dotierter Katalysator verwendet wird.

8. Verfahren nach Anspruch 6 oder Anspruch 7,
**dadurch gekennzeichnet**,
daß das Dotierungsmetall in einer Menge von 0,1 - 50 Gew.-% (wt/wt) bezogen auf das Oxid oder Mischoxid eingesetzt wird.

9. Verfahren nach einem oder mehreren der Ansprüche 6 - 9,
**dadurch gekennzeichnet**,
daß ein mit Ni und/oder Co dotiertes γ-Al₂O₃- oder Co₃O₄ eingesetzt wird.

10. Verfahren gemäß Anspruch 1,
**dadurch gekennzeichnet**,
daß man ein Carbonat oder ein Halogenid der genannten Elemente einsetzt.

11. Verfahren gemäß Anspruch 10,
**dadurch gekennzeichnet**,
daß ein Cobalt- oder Nickelcarbonat, gegebenenfalls in der Hydratform, einsetzt.

12. Verfahren nach einem der vorhergehenden Ansprüche,
**dadurch gekennzeichnet**,
daß der Katalysator in einem Verhältnis von zwischen 0,01 und 30 Gew.-% (wt/wt) bezogen auf α-Isophoron eingesetzt wird.

13. Verfahren nach Anspruch 10,
**dadurch gekennzeichnet**,
daß der Katalysator in einem Verhältnis von zwischen 0,2 und 10 Gew.-% (wt/wt) bezogen auf α-Isophoron eingesetzt wird.

14. Verfahren nach Anspruch 11, .
**dadurch gekennzeichnet**,
daß der Katalysator in einem Verhältnis von zwischen 0,5 und 5 Gew.-% (wt/wt) bezogen auf α-Isophoron eingesetzt wird.

15. Verfahren nach einem oder mehreren der vorhergehenden Ansprüche,
**dadurch gekennzeichnet**,
daß bei Temperaturen zwischen 100 und <300 °C, bevorzugt 150 - 260 °C, isomerisiert wird, wobei der Druck so eingestellt wird, daß die Flüssigphase aufrechterhalten wird.

16. Verfahren nach Anspruch 15,
**dadurch gekennzeichnet**,
daß bei Normaldruck (etw. 1x10⁵ Pa) und der Siedetemperatur des α-Isophorons isomerisiert wird.

17. Verfahren nach einem oder mehreren der vorhergehenden Ansprüche,
**dadurch gekennzeichnet**,
daß die isomerisierte Flüssigphase zur Trennung von α-Isophoron und β-Isophoron destilliert wird.

18. Verfahren nach Anspruch 17,
**dadurch gekennzeichnet**,
daß das Sumpfprodukt der Destillation in die Isomerisierung recycliert wird.

19. Verfahren nach Anspruch 17-,
**dadurch gekennzeichnet**,
daß das Verfahren kontinuierlich betrieben wird.

20. Verfahren nach einem oder mehreren der Ansprüche 1 bis 19,
dadurch gekennzeichnet, daß man die Isomerisierung bei einer Temperatur von 150 bis 260 ° C und einem Druck von 1000 bis 1,5 x 10⁵ Pa durchführt, kontinuierlich Reaktionsgemisch abzieht und dieses bei einem Druck von 100 bis 3 x 10⁴ Pa destilliert und den Destillationssumpf gegebenenfalls in die Isomerisierung zurückführt.

21. Verfahren gemäß Anspruch 20,
dadurch gekennzeichnet, daß man das Reaktionsgemisch mit einer Menge von 5- bis 95 wt% /h abzieht.

## Claims

1. Process for the production of 3,5,5-trimethyl-3-cyclohexen-1-one (β-isophorone) by isomerisation of 3,5,5-trimethyl-2-cyclohexen-1-one (α-isophorone) in the liquid phase in the presence of a heterogeneous catalyst,
characterised in that
an oxide or mixed oxide of an element from groups IIₐ, VIII, I_{b}, IIIₐ and Vₐ of the periodic table, or salts of the above elements that are insoluble under test conditions, ZnO, TiO₂, ZrO₂ or MoO₃, is used as catalyst and the isomerisation is performed without the addition of an organic base.

2. Process according to claim 1,
characterised in that
an oxide or mixed oxide of an element from groups IIₐ and VIII of the periodic table is used.

3. Process according to claim 2,
characterised in that
an oxide or mixed oxide of Ca and/or Mg is used.

4. Process according to claim 2,
characterised in that
an oxide or mixed oxide of Co and/or Ni is used.

5. Process according to claim 4,
characterised in that
Co₃O₄ is used.

6. Process according to one or more of the above claims,
characterised in that
a catalyst is used which is doped with a metal from group VIII of the periodic table.

7. Process according to claim 6,
characterised in that
a catalyst doped with Ni and/or Co is used.

8. Process according to claim 6 or claim 7,
characterised in that
the doping metal is used in a quantity of 0.1 - 50 wt.%, based on the oxide or mixed oxide.

9. Process according to one or more of claims 6 - 9,
characterised in that
a γ-Al₂O₃ or Co₃O₄ doped with Ni and/or Co is used.

10. Process according to claim 1,
characterised in that
a carbonate or a halide of the above elements is used.

11. Process according to claim 10,
characterised in that
a cobalt or nickel carbonate, optionally in the form of the hydrate, is used.

12. Process according to one of the above claims,
characterised in that
the catalyst is used in a ratio of between 0.01 and 30 wt.%, based on α-isophorone.

13. Process according to claim 10,
characterised in that
the catalyst is used in a ratio of between 0.2 and 10 wt.%, based on α-isophorone.

14. Process according to claim 11,
characterised in that
the catalyst is used in a ratio of between 0.5 and 5 wt.%, based on α-isophorone.

15. Process according to one or more of the above claims,
characterised in that
isomerisation is performed at temperatures of between 100 and <300°C, preferably 150 - 260°C, the pressure being set such that the liquid phase is maintained.

16. Process according to claim 15,
characterised in that
isomerisation is performed at standard pressure (approx. 1 x 10⁵ Pa) and at the boiling point of α-isophorone.

17. Process according to one or more of the above claims,
characterised in that
the isomerised liquid phase is distilled to separate α-isophorone and β-isophorone.

18. Process according to claim 17,
characterised in that
the bottom product from the distillation is recycled into the isomerisation.

19. Process according to claim 17,
characterised in that the process is operated continuously.

20. Process according to one or more of claims 1 to 19,
characterised in that
the isomerisation is performed at a temperature of 150 to 260°C and a pressure of 1000 to 1.5 x 10⁵ Pa, reaction mixture is continuously drawn off and is distilled at a pressure of 100 to 3 x 10⁴ Pa and the distillation bottom product is optionally recycled into the isomerisation.

21. Process according to claim 20,
characterised in that
the reaction mixture is drawn off in a quantity of 5 to 95 wt.%/h.

## Revendications

1. Procédé pour la préparation de 3,5,5-triméthylcyclohexa-3-èn-1-one (β-isophorone) par isomérisation de 3,5,5-triméthylcyclohexa-2-èn-1-one (α-isophorone) en phase liquide en présence d'un catalyseur hétérogène, caractérisé en ce qu'on utilise comme catalyseur un oxyde ou un oxyde mixte d'un élément des groupes IIₐ, VIII, I_{b}, IIIₐ et Vₐ du système périodique ou des sels insolubles des éléments mentionnés dans les conditions d'essai, du ZnO, du TiO₂, du ZrO₂ ou du MoO₃ et en ce qu'on réalise l'isomérisation sans addition d'une base organique.

2. Procédé selon la revendication 1,caractérisé en ce qu'on utilise un oxyde ou un oxyde mixte d'un élément des groupes IIₐ et VIII du système périodique.

3. Procédé selon la revendication 2, caractérisé en ce qu'on utilise un oxyde ou un oxyde mixte de Ca et/ou de Mg.

4. Procédé selon la revendication 2, caractérisé en ce qu'on utilise un oxyde ou un oxyde mixte de Co et/ou de Ni.

5. Procédé selon la revendication 4, caractérisé en ce qu'on utilise du Co₃O₄.

6. Procédé selon l'une ou plusieurs des revendications précédentes, caractérisé en ce qu'on utilise un catalyseur qui est dopé avec un métal du groupe VIII du système périodique.

7. Procédé selon la revendication 6, caractérisé en ce qu'on utilise un catalyseur dopé avec du Ni et/ou du Co.

8. Procédé selon la revendication 6 ou la revendication 7, caractérisé en ce que le métal de dopage est utilisé en une quantité de 0,1 à 50 % en poids (P/P) par rapport à l'oxyde ou à l'oxyde mixte.

9. Procédé selon l'une ou plusieurs des revendications 6 à 9, caractérisé en ce qu'on utilise un γ-Al₂O₃ ou un Co₃O₄ dopé avec du Ni et/ou du Co.

10. Procédé selon la revendication 1, caractérisé en ce qu'on utilise un carbonate ou un halogénure des éléments mentionnés.

11. Procédé selon la revendication 10, caractérisé en ce qu'on utilise un carbonate de cobalt ou de nickel, le cas échéant sous forme hydratée.

12. Procédé selon l'une quelconque des revendications précédentes, caractérisé en ce que le catalyseur est utilisé dans un rapport entre 0,01 et 30 % en poids (P/P) par rapport à l'α-isophorone.

13. Procédé selon la revendication 10, caractérisé en ce que le catalyseur est utilisé dans un rapport entre 0,2 et 10 % en poids (P/P) par rapport à l'α-isophorone.

14. Procédé selon la revendication 11, caractérisé en ce que le catalyseur est utilisé dans un rapport entre 0,5 et 5 % en poids (P/P) par rapport à l'α-isophorone.

15. Procédé selon l'une ou plusieurs des revendications précédentes, caractérisé en ce qu'on isomérise à des températures entre 100 et <300 °C, de préférence entre 150 et 260 °C, la pression étant réglée de telle manière que la phase liquide est maintenue.

16. Procédé selon la revendication 15, caractérisé en ce qu'on isomérise sous pression normale (environ 1 x 10⁵ Pa) et à la température d'ébullition de l'α-isophorone.

17. Procédé selon l'une ou plusieurs des revendications précédentes, caractérisé en ce que la phase liquide isomérisée est distillée pour la séparation de l'α-isophorone et de la β-isophorone.

18. Procédé selon la revendication 17, caractérisé en ce que le résidu de distillation est recyclé dans l'isomérisation.

19. Procédé selon la revendication 17, caractérisé en ce que le procédé fonctionne en continu.

20. Procédé selon l'une ou plusieurs des revendications 1 à 19, caractérisé en ce qu'on effectue l'isomérisation à une température de 150 à 260 °C et sous une pression de 1000 à 1,5 x 10⁵ Pa, qu'on retire du mélange réactionnel en continu et qu'on distille celui-ci sous une pression de 100 à 3 x 10⁴ Pa et qu'on recycle le cas échéant le résidu de distillation dans l'isomérisation.

21. Procédé selon la revendication 20, caractérisé en ce qu'on retire le mélange réactionnel en une quantité de 5 à 95 % en poids/ h.
